# EUROPEAN PATENT SPECIFICATION

(11) **EP 2 514 440 B1**
(45) Date of publication and mention of the grant of the patent: **29.03.2017**
(21) Application number: 10837593.2
(22) Date of filing: 14.12.2010
(51) Int. Cl.: A61K 45/06, A61K 9/70, A61K 31/167, A61K 31/245, A61K 31/42, A61K 47/18, A61P 23/02, A61P 29/00, A61K 9/00

(54) **TRANSDERMAL PREPARATION CONTAINING BASIC ANTI-INFLAMMATORY AGENT**
TRANSDERMALES PRÄPARAT MIT EINEM BASISCHEN ENTZÜNDUNGSHEMMENDEN MITTEL
PRÉPARATION TRANSDERMIQUE CONTENANT UN AGENT ANTI-INFLAMMATOIRE BASIQUE

(30) Priority: 15.12.2009 JP 2009284326
(43) Date of publication of application: 24.10.2012
(73) Proprietor: Teikoku Seiyaku Co., Ltd., Higashikagawa-shi, Kagawa 769-2695 (JP)
(72) Inventor: KATAYAMA, Akiko, Higashikagawa-shi Kagawa 769-2695 (JP); INOO, Katsuyuki, Higashikagawa-shi Kagawa 769-2695 (JP)
(74) Representative: Adam, Holger
(86) International application number: PCT/JP2010/072454
(87) International publication number: WO 2011/074567

(56) References cited:
- WO-A1-00/38731
- WO-A1-01/47559
- WO-A1-2005/115355
- WO-A1-2009/066457
- JP-A- 6 040 888
- JP-A- 6 040 947
- JP-A- 9 309 825
- JP-A- 10 507 199
- JP-A- 63 201 119
- JP-A- 2002 128 699
- JP-A- 2005 145 931
- JP-A- 2005 179 319
- JP-A- 2006 509 762
- JP-A- 2009 298 741

## Description

### TECHNICAL FIELD

The present invention relates to a transdermally absorbable preparation, and more particularly, to an adhesive patch containing a basic anti-inflammatory analgesic as a medicinal component and a local anesthetic, which serves not only as the local anesthetic but also as an absorption promoter for the basic anti-inflammatory analgesic.

### BACKGROUND ART

Various attempts have been made to enhance the transdermal absorption of a basic drug in an adhesive patch for many years.

For example, a method for adding a specific solubilizer or absorption promoter in an adhesive patch has been proposed. Patent Document 1 has disclosed a matrix patch in which triacetin is formulated as a permeation enhancer for a basic drug having the dissociation constant (pKa) of eight or more.

Patent Document 2 also has reported an adhesive patch in which a fatty acid having a specific number of carbon atoms is formulated in an adhesive patch to enhance the transdermal absorption of a basic drug.

Further, Patent Documents 3 to 5 also have disclosed adhesive patches in which a basic drug and an organic acid and/or an organic salt are formulated.

In these proposals, the skin permeability of the basic drug has been enhanced by formation of a stable ion pair between the basic drug and the organic acid (salt) formulated.

However, many of additives to be formulated in these adhesive patches have damaged the physical properties of the adhesive patches as they are formulated, thereby resulting in a drawback in that these additives cannot be formulated in a large amount. In addition, many of such additives also have had high skin irritation.

On the other hand, an attempt to use a local anesthetic as an absorption promoter for various drugs also has been made, and many reports have been related to an adhesive patch in which a non-steroidal anti-inflammatory analgesic and the local anesthetic are formulated (Patent Documents 6 to 10).

The drugs used in these reports, however, are the adhesive patches in which an acidic drug such as indomethacin, diclofenac sodium and loxoprofen sodium is formulated and, in fact, few reports have been related to an adhesive patch in which a basic anti-inflammatory analgesic such as acetaminophen, butorphanol and buprenorphine is formulated with the local anesthetic.

Accordingly, as for an adhesive patch in which a basic anti-inflammatory analgesic and a local anesthetic as a transdermal absorption promoter are formulated, it has been desired to develop a transdermally absorbable preparation which achieves high anti-inflammatory and analgesic effects without inhibiting drug releasing of each other.

JP 63-201119 A discloses a plaster composition containing, as active components, (A) a solid drug insoluble in water and (B) one or more substances capable of dissolving said drug and selected from benzyl alcohol, crotamiton, diphenhydramine, nicotinic acid ester, salicylic acid ester and a mixture of menthol and camphor.

JP 09-309825 A describes a percutaneous patch material comprising (A) an adhesive layer containing (i) a medicine and (ii) a compound having an isoprenoid skeleton of a specific formula and (B) a supporter.

JP 2006 509762 A discloses a selective cyclooxygenase-2 inhibitor patch.

WO 01/47559 A1 discloses patches for external use, in particular an adhesive patch comprising 0.6% of bufexamac and 8% of xylocaine.

### PRIOR ART DOCUMENTS

### PATENT DOCUMENTS

Patent Document 1: Japanese Translation of PCT International Application No. Hei. 10-507199
Patent Document 2: Japanese Patent Application Laid-Open No. 2009-242303
Patent Document 3: International Publication No. WO 00/061120
Patent Document 4: International Publication No. WO 01/007018
Patent Document 5: International Publication No. WO 2005/115355
Patent Document 6: Japanese Patent Application Laid-Open No. 2002-128699
Patent Document 7: Japanese Patent Application Laid-Open No. 2003-335663
Patent Document 8: Japanese Patent Application Laid-Open No. 2004-123632
Patent Document 9: Japanese Patent Application Laid-Open No. 2005-145931
Patent Document 10: Japanese Patent Application Laid-Open No. 2005-145932
Patent Document 11: JP 63-201119 A
Patent Document 12: JP 09-309825 A
Patent Document 13: JP 2006 509762 A
Patent Document 14: WO 01/47559 A1

Under such circumstances, when a basic anti-inflammatory analgesic is formulated with a transdermal absorption promoter, the present inventors have intensively studied development of the transdermally absorbable preparation, which achieves high anti-inflammatory and analgesic effects without inhibiting the drug releasing of each other.

As a result, by selecting a local anesthetic as the absorption promoter for the basic anti-inflammatory analgesic and formulating it in an adhesive patch base, it was found that the transdermally absorbable preparation having excellent releasing of the basic anti-inflammatory analgesic was obtained while it had excellent analgesic activity at the same time without losing the transdermal absorption of the local anesthetic, thereby completing the present invention.

### SUMMARY OF THE INVENTION

### PROBLEMS TO BE SOLVED BY THE INVENTION

Therefore, the present invention is to solve the above-mentioned conventional problems and, for a transdermally absorbable preparation in which the basic anti-inflammatory analgesic is formulated, it is a first object of the present invention to provide an external adhesive patch having excellent drug releasing without damaging the physical properties of the preparation.

Further, for a transdermally absorbable preparation in which a local anesthetic and a basic anti-inflammatory analgesic are formulated, it is a second object of the present invention to provide a preparation, which may have high releasing of the basic anti-inflammatory analgesic without losing the releasing of the local anesthetic.

### MEANS FOR SOLVING THE PROBLEM

The basic aspect of the present invention to solve such problems is a transdermally absorbable adhesive patch, which contains both a basic anti-inflammatory analgesic and a local anesthetic as an absorption promoter for the basic anti-inflammatory analgesic, wherein the basic anti-inflammatory analgesic has the acid dissociation constant (pKa) of 7 or more and is selected from the group consisting of acetaminophen, butorphanol tartrate, buprenorphine hydrochloride, epirizole, celecoxib, and valdecoxib.

Specifically, the present invention is the transdermally absorbable adhesive patch wherein the content of the basic anti-inflammatory analgesic is from 0.1% to 10% by weight to the total weight of the drug-containing plaster base material and the content of the absorption promoter is from 0.01% to 20% by weight to the total weight of the drug-containing plaster base material.

Most specifically, the present invention is the transdermally absorbable adhesive patch wherein the basic anti-inflammatory analgesic is acetaminophen or valdecoxib, and the local anesthetic is lidocaine or oxybuprocaine.

### EFFECTS OF THE INVENTION

The present invention provides a transdermally absorbable adhesive patch, which contains both a basic anti-inflammatory analgesic and a local anesthetic as an absorption promoter for the basic anti-inflammatory analgesic.

Particularly, by configuring the adhesive patch in which the local anesthetic is formulated with the basic anti-inflammatory analgesic in the plaster composition, the present invention can provide the transdermally absorbable preparation, which has high releasing of the basic anti-inflammatory analgesic and also may have excellent analgesic effect of the local anesthetic.

Accordingly, the present invention has a great medical effect in that the adhesive patch of the transdermally absorbable preparation containing clinically extremely useful basic anti-inflammatory analgesic can be provided.

### BRIEF DESCRIPTION OF DRAWINGS

Fig. 1 is a graph showing the result of in vitro rat skin permeability test for acetaminophen by using the acetaminophen/lidocaine formulated preparation (Example 1) of the present invention in Comparative Study (1).
Fig. 2 is a graph showing the result of in vitro rat skin permeability test for lidocaine by using the acetaminophen/lidocaine formulated preparation (Example 1) of the present invention in Comparative Study (1).
Fig. 3 is a graph showing the result of in vitro rat skin permeability test for acetaminophen by using the acetaminophen/oxybuprocaine formulated preparation (Example 2) of the present invention in Comparative Study (2).
Fig. 4 is a graph showing the result of in vitro rat skin permeability test for oxybuprocaine by using the acetaminophen/oxybuprocaine formulated preparation (Example 2) of the present invention in Comparative Study (2).
Fig. 5 is a graph showing the result of in vitro rat skin permeability test for valdecoxib by using the valdecoxib/oxybuprocaine formulated preparation (Example 3) of the present invention in Comparative Study (3).
Fig. 6 is a graph showing the result of in vitro rat skin permeability test for oxybuprocaine by using the valdecoxib/oxybuprocaine formulated preparation (Example 3) of the present invention in Comparative Study (3).

### EMBODIMENTS FOR CARRYING OUT THE INVENTION

The basic aspect of the present invention, as described above, is the transdermally absorbable adhesive patch, which contains both a basic anti-inflammatory analgesic and a local anesthetic as an absorption promoter for the basic anti-inflammatory analgesic.

The basic anti-inflammatory analgesic used in the transdermally absorbable adhesive patch provided by the present invention has the acid dissociation constant (pKa) of 7 or more and the basic anti-inflammatory analgesic is selected from the group consisting of acetaminophen, butorphanol tartrate, buprenorphine hydrochloride, epirizole, celecoxib, and valdecoxib. In particular, in the use of acetaminophen and valdecoxib, the effect thereof was high.

In this case, the formulated amount of the basic anti-inflammatory analgesic is preferably from 0.1% to 10% by weight and particularly preferably from 0.2% to 5% by weight to the total weight of the drug-containing plaster base material, i.e., in the plaster composition.

When the formulated amount of the drug is less than 0.1%, the medicinal effect of the anti-inflammatory analgesic may not be sufficient. Ten% or more of the drug formulated is not preferable because the physical properties of the plaster may be lost.

On the other hand, in the present invention, the local anesthetic to be formulated together with basic anti-inflammatory analgesic can be formulated without any problems as long as it is well-known, but particularly lidocaine or oxybuprocaine is preferable.

These local anesthetics not only have the analgesic activity in themselves, but also serve as the absorption promoter for the basic anti-inflammatory analgesic in the present invention.

In such a case, the formulated amount of the local anesthetic is preferably from 0.01% to 20% by weight and more preferably from 0.1% to 10% by weight to the total weight of the drug-containing plaster base material.

The formulated amount of less than 0.01% by weight of the local anesthetic neither can enhance the skin permeability of the basic anti-inflammatory analgesic sufficiently, nor may result in sufficient medicinal effect of the local anesthetic. On the other hand, more than 20% by weight of the local anesthetic formulated is also not preferable because not only the effect caused by formulating it cannot be expected, but also irritation to the skin may be caused or the physical properties of the plaster may be lost.

In the adhesive patch provided by the present invention, both the local anesthetic and the basic anti-inflammatory analgesic formulated in the plaster composition achieve the effects such that excellent drug releasing of the basic anti-inflammatory analgesic can be obtained without inhibiting the drug releasing of the local anesthetic.

Among them, in particular, lidocaine or oxybuprocaine is selected as the local anesthetic and acetaminophen is selected as the basic anti-inflammatory analgesic. When combination of these is formulated, very high effect can be obtained.

The plaster composition used in the adhesive patch provided by the present invention can be prepared by mixing the local anesthetic and the basic anti-inflammatory analgesic with the adhesive patch base component.

Such an adhesive patch base component is not particularly limited as long as it can become the base of an adhesive layer which is the plaster composition, and hydrophobic polymers such as a rubber polymer, an acrylic polymer and a silicon polymer are preferably used.

Examples of the rubber polymer may include a styrene-isoprene-styrene block copolymer (hereinafter, referred to as SIS), polyisobutylene (hereinafter, referred to as PIB), a styrene-butadiene-styrene block copolymer (hereinafter, referred to as SBS), a styrene-butadiene rubber (hereinafter, referred to as SBR), an isoprene rubber and the like. Among them, SIS is particularly preferred.

Also, the acrylic polymer is not particularly limited as long as one of (meth)acrylic acid derivatives represented by 2-ethylhexyl acrylate, methyl acrylate, butyl acrylate, hydroxyethyl acrylate, 2-ethylhexyl methacrylate and the like is contained and copolymerized. For example, the adhesives listed in Japanese Pharmaceutical Excipients Directory 2007 (edited by International Pharmaceutical Excipients Council Japan) such as the adhesive of an acrylic polymer which contains an acrylic acid/octyl acrylate copolymer, a 2-ethylhexyl acrylate/vinylpyrrolidone copolymer solution, an acrylate-vinyl acetate copolymer, a 2-ethylhexyl acrylate-2-ethylhexyl methacrylate/dodecyl methacrylate copolymer, a methyl acrylate-2-ethylhexyl acrylate copolymer resin emulsion, an acrylic resin alkanol amine solution and the like, DURO-TAK acrylic adhesive series (produced by National Starch and Chemical Company) and Eudragit series (HIGUCHI Inc.) can be used.

Moreover, specific examples of the silicon polymer may include a silicone rubber such as polyorganosiloxane.

Such hydrophobic polymers may be used in mixture of two or more. The formulated amount of such polymers based on the mass of the total composition is from 5% to 80% by weight, preferably from 10% to 70% by weight and more preferably from 10% to 50% by weight in consideration of the formation of the adhesive layer and sufficient drug permeability.

The adhesive composition in the adhesive patch which is the transdermally absorbable preparation provided by the present invention may contain a plasticizer. Examples of the plasticizer to be used may include a petroleum-based oil (for example, a paraffin-based process oil such as a liquid paraffin, a naphthene-based process oil, an aromatic process oil and the like), squalane, squalene, a vegetable oil (for example, an olive oil, a camellia oil, a tall oil, a peanut oil, a castor oil and the like), a silicone oil, dibasic acid ester (for example, dibutyl phthalate, dioctyl phthalate and the like), a liquid rubber (for example, polybutene, a liquid isoprene rubber and the like), liquid fatty acid esters (for example, isopropyl myristate, hexyl laurate, diethyl sebacate, diisopropyl sebacate and the like). A liquid paraffin is particularly preferred.

Such components may be used in mixture of two or more. The formulated amount of such plasticizers based on the total composition of the adhesive layer is from 1% to 70% by weight, preferably from 10% to 60% by weight and more preferably from 10% to 50% by weight in total in consideration of the maintaining of enough cohesion as the adhesive patch.

In the adhesive layer of the present invention, it is desirable to formulate a tackifier resin to adjust the adhesion of the preparation. Examples of the tackifier resin which can be used may include rosin derivatives (for example, rosin, rosin glycerin ester, hydrogenated rosin, hydrogenated rosin glycerin ester, rosin pentaerythritol ester and the like), an alicyclic saturated hydrocarbon resin (for example, Alcon P100, Arakawa Chemical Industries Ltd.), an aliphatic hydrocarbon resin (for example, Quinton B170, Nippon Zeon Co., Ltd.), a terpene resin (for example, Clearon P-125, Yasuhara Chemical Co., Ltd.), a maleic acid resin and the like.

The formulated amount of such a tackifier resin based on the total composition of the adhesive composition can be from 5% to 70% by weight, preferably from 5% to 60% by weight and more preferably from 10% to 50% by weight in consideration of enough adhesion as the adhesive preparation and irritation to the skin upon being peeled.

Also, an antioxidant, a filler, a cross-linking agent, a preservative and an ultraviolet absorber can be used if necessary. As the antioxidant, tocopherol and ester derivatives thereof, ascorbic acid, ascorbyl stearate, nordihydroguaiaretic acid, dibutylhydroxytoluene (hereinafter, referred to as BHT), butylhydroxyanisole and the like are desirable.

As the filler, calcium carbonate, magnesium carbonate, silicate (for example, aluminum silicate, magnesium silicate and the like), silicic acid, barium sulfate, calcium sulfate, calcium zincate, zinc oxide, titanium oxide and the like are desirable.

As the cross-linking agent, a thermosetting resin such as an amino resin, a phenolic resin, an epoxy resin, an alkyd resin and unsaturated polyester; an isocyanate compound; a blocked isocyanate compound; an organic cross-linking agent; and an inorganic cross-linking agent such as a metal and a metal compound are desirable.

As the preservative, paraben such as ethyl parahydroxybenzoate, propyl parahydroxybenzoate and butyl parahydroxybenzoate is desirable.

As the ultraviolet absorber, p-aminobenzoic acid derivatives, anthranilic acid derivatives, salicylic acid derivatives, amino acid compounds, dioxane derivatives, coumarin derivatives, imidazoline derivatives, pyrimidine derivatives and the like are desirable.

Such an antioxidant, a filler, a cross-linking agent, a preservative and an ultraviolet absorber can be formulated in 10% by weight or less, preferably 5% by weight or less and more preferably 2% by weight or less based on the mass of the total composition of the adhesive layer of the preparation.

The adhesive patch, which is the transdermally absorbable preparation of the present invention having the composition described above, can be produced by any methods.

Examples of the methods include generally called a hot melt method and a solvent method. In the hot melt method, the adhesive patch can be obtained by thermally melting the drug-containing base component, coating it on a release film or a support, and laminating the base component to a support or a release film. In the solvent method, the adhesive patch can be obtained by dissolving the drug-containing base component in an organic solvent such as toluene, hexane, ethyl acetate or N-methyl-2-pyrrolidone, spreading and coating it on a release film or a support, removing the solvent by drying, and laminating the base component to a support or a release film.

In the adhesive patch which is the external transdermal preparation provided by the present invention, the thickness of the adhesive layer is not particularly limited, but generally 500 µm or less and preferably from 20 µm to 300 µm.

As the support of the adhesive patch which is the transdermally absorbable preparation of the present invention, an elastic or a non-elastic support can be used. For example, it is selected from fabrics, nonwoven fabrics, polyurethane, polyester, polyvinyl acetate, polyvinylidene chloride, polyethylene, polyethylene terephthalate (hereinafter, referred to as PET), an aluminum sheet and the like, or the composite material thereof.

The release film is not particularly limited as long as it protects the adhesive layer without the main drug components being decomposed until the adhesive patch, which is the transdermally absorbable preparation, is applied to the skin and it is silicon coated to be easily peeled. Specific examples of the release film include a silicon coated polyethylene film, PET film and polypropylene film.

### EXAMPLE

Hereinafter, the present invention will be described more specifically by illustrating Examples, Preparation Examples and Test Examples of the present invention, but the present invention is not limited to these Examples and Preparation Examples and various modifications thereof can be made without departing from the technical idea of the present invention.

Here, in the following description, all of "%" mean "% by weight" unless otherwise specified.

### Example 1: acetaminophen / lidocaine formulated preparation

Acetaminophen was selected as a basic anti-inflammatory analgesic and lidocaine was selected as a local anesthetic. The external adhesive patch in which both acetaminophen and lidocaine were formulated was prepared.

| (Components) | |
|---|---|
| SIS | 16% |
| Liquid paraffin | 28% |
| BHT | 1% |
| Hydrogenated rosin glycerin ester | 40% |
| Lidocaine | 10% |
| Acetaminophen | 5% |
| Total | 100% |

### (Process)

Acetaminophen was dissolved in advance in N-methyl-2-pyrrolidone and lidocaine was dissolved in toluene. They were mixed with other base components which were already dissolved in toluene. The mixture was coated on the release film, and subsequently toluene and N-methyl-2-pyrrolidone were removed by drying. The obtained product was laminated with the PET film support to provide a desirable transdermally absorbable preparation (the thickness of the adhesive layer was 100 µm).

### Example 2: acetaminophen / oxybuprocaine formulated preparation

Acetaminophen was selected as a basic anti-inflammatory analgesic and oxybuprocaine was selected as a local anesthetic. The external adhesive patch in which both acetaminophen and oxybuprocaine were formulated was prepared.

| (Components) | |
|---|---|
| SIS | 16% |
| Liquid paraffin | 28% |
| BHT | 1% |
| Hydrogenated rosin glycerin ester | 40% |
| Oxybuprocaine | 10% |
| Acetaminophen | 5% |
| Total | 100% |

### (Process)

Acetaminophen was dissolved in advance in N-methyl-2-pyrrolidone and oxybuprocaine was dissolved in toluene. They were mixed with other base components which were already dissolved in toluene. The mixture was coated on the release film, and subsequently toluene and N-methyl-2-pyrrolidone were removed by drying. The obtained product was laminated with the PET film support to provide a desirable transdermally absorbable preparation (the thickness of the adhesive layer was 100 µm).

### Example 3: valdecoxib / oxybuprocaine formulated preparation

Valdecoxib was selected as a basic anti-inflammatory analgesic and oxybuprocaine was selected as a local anesthetic. The external adhesive patch in which both valdecoxib and oxybuprocaine were formulated was prepared.

| (Components) | |
|---|---|
| SIS | 18% |
| Liquid paraffin | 23% |
| BHT | 1% |
| Hydrogenated rosin glycerin ester | 40% |
| Oxybuprocaine | 15% |
| Valdecoxib | 3% |
| Total | 100% |

### (Process)

Valdecoxib was dissolved in advance in N-methyl-2-pyrrolidone and oxybuprocaine was dissolved in toluene. They were mixed with other base components which were already dissolved in toluene. The mixture was coated on the release film, and subsequently toluene and N-methyl-2-pyrrolidone were removed by drying. The obtained product was laminated with the PET film support to provide a desirable transdermally absorbable preparation (the thickness of the adhesive layer was 100 µm).

### Comparative Example 1: acetaminophen formulated preparation

The external adhesive patch in which only acetaminophen was formulated was prepared as Comparative Example 1.

| (Components) | |
|---|---|
| SIS | 16% |
| Liquid paraffin | 38% |
| BHT | 1% |
| Hydrogenated rosin glycerin ester | 40% |
| Acetaminophen | 5% |
| Total | 100% |

### (Process)

Acetaminophen was dissolved in advance in N-methyl-2-pyrrolidone, and the solution was mixed with other base components which were already dissolved in toluene. The mixture was coated on the release film, and subsequently toluene and N-methyl-2-pyrrolidone were removed by drying. The obtained product was laminated with the PET film support to provide a desirable transdermally absorbable preparation (the thickness of the adhesive layer was 100 µm).

### Comparative Example 2: lidocaine formulated preparation

The external adhesive patch in which only lidocaine was formulated was prepared as Comparative Example 2.

| (Components) | |
|---|---|
| SIS | 16% |
| Liquid paraffin | 33% |
| BHT | 1% |
| Hydrogenated rosin glycerin ester | 40% |
| Lidocaine | 10% |
| Total | 100% |

### (Process)

Lidocaine and other base components were dissolved and mixed in toluene. The mixture was coated on the release film, and subsequently toluene was removed by drying. The obtained product was laminated with the PET film support to provide a desirable transdermally absorbable preparation (the thickness of the adhesive layer was 100 µm).

### Comparative Example 2: oxybuprocaine formulated preparation

The external adhesive patch in which only oxybuprocaine was formulated was prepared as Comparative Example 3.

| (Components) | |
|---|---|
| SIS | 16% |
| Liquid paraffin | 33% |
| BHT | 1% |
| Hydrogenated rosin glycerin ester | 40% |
| Oxybuprocaine | 10% |
| Total | 100% |

### (Process)

Oxybuprocaine and other base components were dissolved and mixed in toluene. The mixture was coated on the release film, and subsequently toluene was removed by drying. The obtained product was laminated with the PET film support to provide a desirable transdermally absorbable preparation (the thickness of the adhesive layer was 100 µm).

### Comparative Example 4: valdecoxib formulated preparation

The external adhesive patch in which only valdecoxib was formulated was prepared as Comparative Example 4.

| (Components) | |
|---|---|
| SIS | 16% |
| Liquid paraffin | 38% |
| BHT | 1% |
| Hydrogenated rosin glycerin ester | 40% |
| Valdecoxib | 3% |
| Total | 100% |

### (Process)

Valdecoxib was dissolved in advance in N-methyl-2-pyrrolidone, and the solution was mixed with other base components which were already dissolved in toluene. The mixture was coated on the release film, and subsequently toluene was removed by drying. The obtained product was laminated with the PET film support to provide a desirable transdermally absorbable preparation (the thickness of the adhesive layer was 100 µm).

### Comparative Example 5: oxybuprocaine formulated preparation

The external adhesive patch in which only oxybuprocaine was formulated was prepared as Comparative Example 5.

| (Components) | |
|---|---|
| SIS | 18% |
| Liquid paraffin | 26% |
| BHT | 1% |
| Hydrogenated rosin glycerin ester | 40% |
| Oxybuprocaine | 15% |
| Total | 100% |

### (Process)

Oxybuprocaine and other base components were dissolved and mixed in toluene. The mixture was coated on the release film, and subsequently toluene was removed by drying. The obtained product was laminated with the PET film support to provide a desirable transdermally absorbable preparation (the thickness of the adhesive layer was 100 µm).

Hereinafter, the tests for Comparative Studies (1) to (3) were carried out by using Examples and Comparative Examples.

### [Comparative Study]

(1) A study for releasing of acetaminophen and lidocaine in comparison of the preparation of Example 1 in which both acetaminophen and lidocaine are formulated in the external adhesive patch of the present invention, the preparation of Comparative Example 1 in which only acetaminophen is formulated, and the preparation of Comparative Example 2 in which only lidocaine is formulated.
(2) A study for releasing of acetaminophen and oxybuprocaine in comparison of the preparation of Example 2 in which both acetaminophen and oxybuprocaine which is another local anesthetic are formulated in the external adhesive patch of the present invention, the preparation of Comparative Example 1 in which only acetaminophen is formulated, and the preparation of Comparative Example 3 in which only oxybuprocaine is formulated.
(3) A study for releasing of valdecoxib and oxybuprocaine in comparison of the preparation of Example 3 in which both oxybuprocaine and valdecoxib which is another basic anti-inflammatory analgesic are formulated in the external adhesive patch of the present invention, the preparation of Comparative Example 4 in which only valdecoxib is formulated, and the preparation of Comparative Example 5 in which only oxybuprocaine is formulated.

### Test Example 1: Rat Skin Permeability Test

In vitro skin permeability test was carried out with the skin excised from the male rat (Wister strain, 8 week old) for each external adhesive patch prepared in the above Example 1 and Example 2, and Comparative Examples 1 to 3 to study the specificity of the releasing of respective drugs in the external adhesive patch of the present invention in which both the local anesthetic and the basic anti-inflammatory analgesic were formulated.

### [Method]

The rat abdominal skin was exfoliated, the dermis side of the skin was directed to a side of a receptor layer, and its inside was filled with phosphate buffered saline. Water kept at 37°C was circulated in a water jacket. Each test preparation prepared in Example 1, Example 2 and Comparative Examples 1 to 3 was stamped out in a circle (1.77 cm²) and attached to the excised skin. The receptor solution was sampled over time to measure the permeated amount of each drug (lidocaine, oxybuprocaine, and aminoacetophen) by high performance liquid chromatography.

### Test Example 2: Rat Skin Permeability Test

In vitro skin permeability test was carried out with the skin excised from the male hairless rat (HWY strain, 7 week old) for each external adhesive patch prepared in the above Example 3 and Comparative Examples 4 to 5 to study the specificity of the releasing of respective drugs in the external adhesive patch of the present invention in which both the local anesthetic and the basic anti-inflammatory analgesic were formulated.

### [Method]

The rat abdominal skin was exfoliated, the dermis side of the skin was directed to a side of a receptor layer, and its inside was filled with phosphate buffered saline. Water kept at 37°C was circulated in a water jacket. Each preparation prepared in Example 1, Example 3 or Comparative Examples 4 to 5 was stamped out in a circle (1.77 cm²) and attached to the excised skin. The receptor solution was sampled over time to measure the permeated amount of each drug (oxybuprocaine and valdecoxib) by high performance liquid chromatography.

### [Result]

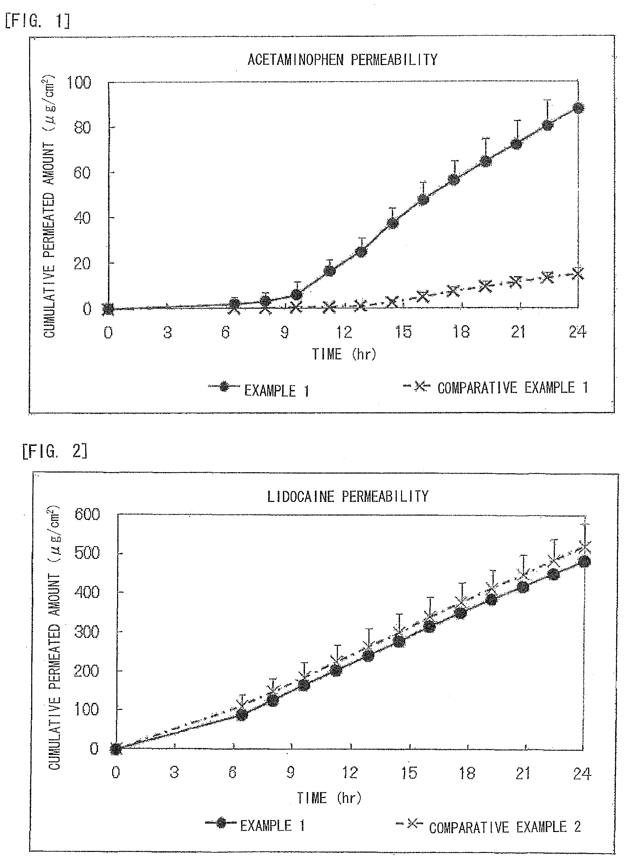

The results are shown in Figs. 1 to 6.

### [Consideration]

### Consideration to Comparative Study (1)

As is apparent in comparison of the results shown in Figs. 1 and 2, the releasing of acetaminophen in the external adhesive patch of Example 1 in which both acetaminophen and lidocaine were formulated was dramatically high compared to the external adhesive patch of Comparative Example 1 in which only acetaminophen was formulated (Fig. 1).

Also, the releasing of lidocaine in the external adhesive patch of Example 1 in which both acetaminophen and lidocaine were formulated was almost the same as that of the external adhesive patch of Comparative Example 2 in which only lidocaine was formulated (Fig. 2)

Considering these both results, it is understood that good releasing of lidocaine as the absorption promoter enhances the releasing of acetaminophen in the external adhesive patch of the present invention in which both acetaminophen and lidocaine are formulated.

### Consideration to Comparative Study (2)

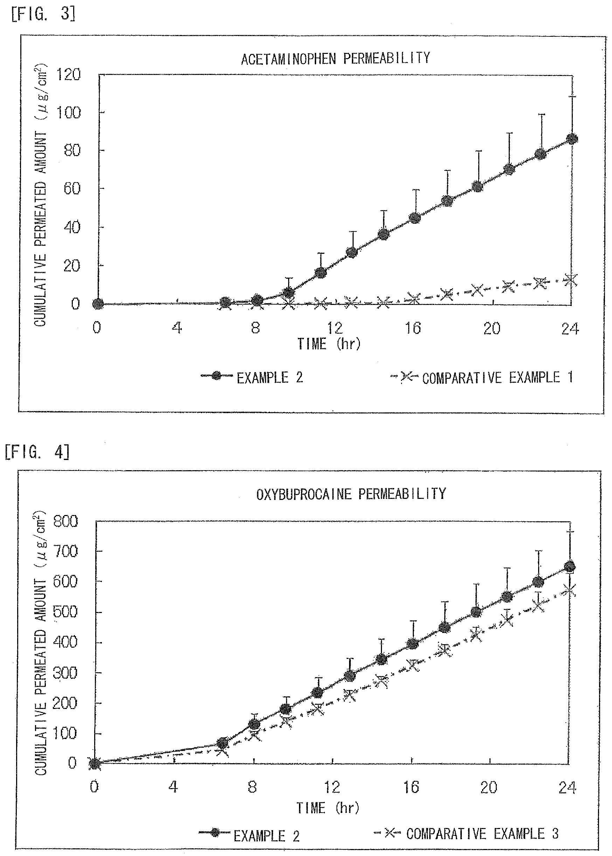

On the other hand, oxybuprocaine, which is another local anesthetic, was subjected to the same test. As in Comparative Study (1), the external adhesive patch of Example 2 in which both acetaminophen and oxybuprocaine were formulated had extremely high releasing of acetaminophen compared to the external adhesive patch of Comparative Example 2 which was the preparation in which only acetaminophen was formulated (Fig. 3).

Further, the external adhesive patch of Example 2 in which both acetaminophen and oxybuprocaine were formulated had almost the same releasing of oxybuprocaine as the external adhesive patch of Comparative Example 3 which was the preparation in which only oxybuprocaine was formulated (Fig. 4).

Thus, even in this case, it is understood that good releasing of oxybuprocaine as the absorption promoter enhances the releasing of acetaminophen in the external adhesive patch of the present invention in which both acetaminophen and oxybuprocaine are formulated.

### Consideration to Comparative Study (3)

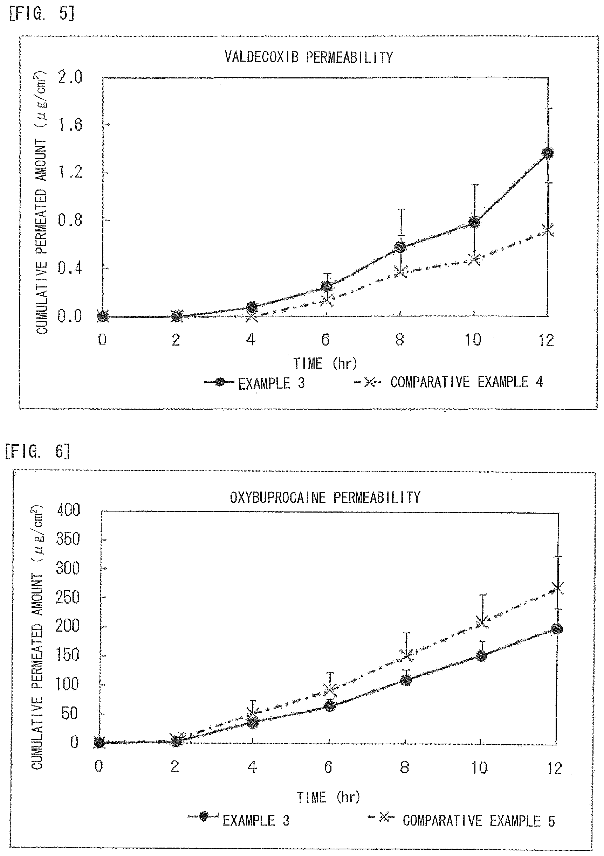

Furthermore, valdecoxib, which is another basic anti-inflammatory analgesic, was subjected the same test. As in Comparative Study (1), the external adhesive patch of Example 3 in which both valdecoxib and oxybuprocaine were formulated had good releasing of valdecoxib compared to the external adhesive patch of Comparative Example 4 which was the preparation in which only valdecoxib was formulated (Fig. 5).

Also, the external adhesive patch of Example 3 in which both valdecoxib and oxybuprocaine were formulated had almost the same releasing of oxybuprocaine as the external adhesive patch of Comparative Example 5 which was the preparation in which only oxybuprocaine was formulated (Fig. 6).

Thus, as in the results of Comparative Studies (1) and (2), it is understood that, even in this case, good releasing of oxybuprocaine as the absorption promoter enhances the releasing of valdecoxib in the external adhesive patch of the present invention in which both valdecoxib and oxybuprocaine are formulated.

According to the results of these Comparative Studies (1) to (3), for the adhesive patch of the present invention in which both the local anesthetic and the basic anti-inflammatory analgesic are formulated, it was found that the adhesive patch is the transdermally absorbable preparation in which the releasing of the local anesthetic is not inhibited and is accompanied by very excellent releasing of the basic anti-inflammatory analgesic. Therefore, extremely excellent specificity of the present invention is to be understood.

### [Preparation Example]

Hereinafter, specific Preparation Examples other than the external adhesive patch of the present invention described above in Examples 1 and 2 are shown below in Tables 1 and 2. Here, the present invention is not limited thereto.

**[Table 1]**

| Components | Preparation Example (unit: % by weight) | | | | | |
|---|---|---|---|---|---|---|
| | 1 | 2 | 3 | 4 | 5 | 6 |
| SIS | 16 | 16 | 15 | 19 | 18 | 25 |
| BHT | 1 | 1 | 1 | 1 | 1 | 1 |
| Hydrogenated Rosin Glycerin Ester | 40 | 40 | | 40 | 40 | |
| Terpene Resin | | | | | | 40 |
| Alicyclic Saturated Hydrocarbon Resin | | | 42 | | | |
| Liquid Paraffin | 32 | 33 | 35 | 36 | 26 | 13 |
| Oxybuprocaine | | | | | 12 | 20 |
| Lidocaine | 10 | 5 | 4 | 5 | | |
| Acetaminophen | 1 | 5 | 2 | 2 | 3 | 1 |

| | | | | | | |
|---|---|---|---|---|---|---|
| The thickness of the adhesive layer: 100 µm | | | | | | |

**[Table 2]**

| Components | Preparation Example (unit: % by weight) | | | | | |
|---|---|---|---|---|---|---|
| | 7 | 8 | 9 | 10 | 11 | 12 |
| SIS | 18 | 20 | 16 | 18 | 18 | 18 |
| BHT | 1 | 1 | 1 | 1 | 1 | 1 |
| Hydrogenated Rosin Glycerin Ester | 40 | 40 | | 40 | 40 | 40 |
| Terpene Resin | | | | | | |
| Alicyclic Saturated Hydrocarbon Resin | | | 40 | | | |
| Liquid Paraffin | 23 | 18.5 | 31 | 25 | 23 | 25 |
| Oxybuprocaine | 15 | 20 | | 15 | 15 | 15 |
| Lidocaine | | | 10 | | | |
| Epirizole | 3 | 0.5 | | | | |
| Butorphanol | | | 2 | | | |
| Celecoxib | | | | 1 | 3 | |
| Valdecoxib | | | | | | 1 |

| | | | | | | |
|---|---|---|---|---|---|---|
| The thickness of the adhesive layer: 100 µm | | | | | | |

### INDUSTRIAL APPLICABILITY

As described above, the transdermally absorbable preparation according to the present invention can provide the preparation which has excellent analgesic effect caused by the local anesthetic and excellent anti-inflammatory and analgesic effects caused by the basic anti-inflammatory analgesic.

Particularly, the present invention can provide the transdermally absorbable preparation, which has high releasing of the basic anti-inflammatory analgesic without losing the releasing of the local anesthetic, thereby achieving excellent anti-inflammatory and analgesic effects. Thus, the present invention has a great medical effect.

## Claims

1. A transdermally absorbable adhesive patch, comprising both a basic anti-inflammatory analgesic having an acid dissociation constant (pKa) of 7 or more which is selected from the group consisting of acetaminophen, butorphanol tartrate, buprenorphine hydrochloride, epirizole, celecoxib, and valdecoxib, and a local anesthetic as an absorption promoter for the basic anti-inflammatory analgesic.

2. The transdermally absorbable adhesive patch according to claim 1, wherein a content of the basic anti-inflammatory analgesic is from 0.1% to 10% by weight to a total weight of a drug-containing plaster.

3. The transdermally absorbable adhesive patch according to claim 1, wherein a content of the absorption promoter is from 0.01% to 20% by weight to a total weight of a drug-containing plaster.

4. The transdermally absorbable adhesive patch according to any of claims 1 to 3, wherein the basic anti-inflammatory analgesic is acetaminophen or valdecoxib.

5. The transdermally absorbable adhesive patch according to any of claims 1 to 3, wherein the local anesthetic is lidocaine or oxybuprocaine.

## Patentansprüche

1. Transdermales absorbierbares adhäsives Patch, umfassend sowohl ein basisches anti-inflammatorisches analgetisches Mittel mit einer Säuredissoziationskonstante (pKa) von 7 oder mehr, ausgewählt aus der Gruppe, bestehend aus Acetaminophen, Butorphanoltartrat, Buprenorphinhydrochlorid, Epirizol, Celecoxib und Valdecoxib und ein Lokalanästhetikum als Absorptionspromotor für das basische anti-inflammatorische analgetische Mittel.

2. Transdermales absorbierbares adhäsives Patch, wobei ein Gehalt des basischen anti-inflammatorischen Analgetikums von 0,1 Gew.-% bis 10 Gew.-%, bezogen auf das Gesamtgewicht eines Arzneimittel-enthaltenden Pflasters, beträgt.

3. Transdermales absorbierbares adhäsives Patch gemäß Anspruch 1, wobei ein Gehalt des Absorptionspromotors von 0,01 Gew.-% bis 20 Gew.-%, bezogen auf das Gesamtgewicht eines Arzneimittel-enthaltenden Pflasters, beträgt.

4. Transdermales absorbierbares adhäsives Patch nach einem der Ansprüche 1 bis 3, wobei das basische antiinflammatorische Analgetikum Acetaminophen oder Valdecoxib ist.

5. Transdermales absorbierbares adhäsives Patch nach einem der Ansprüche 1 bis 3, wobei das Lokalanästhetikum Lidocain oder Oxybuprocain ist.

## Revendications

1. Patch adhésif absorbable par voie transdermique, comprenant à la fois un analgésique anti-inflammatoire basique ayant une constante de dissociation acide (pKa) égale ou supérieure à 7 qui est sélectionné parmi le groupe constitué de l'acétaminophène, du tartrate de butorphanol, du chlorhydrate de buprénorphine, de l'épirizole, du célécoxib, et du valdécoxib, et un anesthésiant local en tant que promoteur d'absorption pour l'analgésique anti-inflammatoire basique.

2. Patch adhésif absorbable par voie transdermique selon la revendication 1, dans lequel une teneur de l'analgésique anti-inflammatoire basique va de 0,1 % à 10 % en poids en fonction d'un poids total d'un emplâtre contenant un médicament.

3. Patch adhésif absorbable par voie transdermique selon la revendication 1, dans lequel une teneur du promoteur d'absorption va de 0,01 % à 20 % en poids en fonction d'un poids total d'un emplâtre contenant un médicament.

4. Patch adhésif absorbable par voie transdermique selon l'une quelconque des revendications 1 à 3, dans lequel l'analgésique anti-inflammatoire basique est l'acétaminophène ou le valdécoxib.

5. Patch adhésif absorbable par voie transdermique selon l'une quelconque des revendications 1 à 3, dans lequel l'anesthésiant local est la lidocaïne ou l'oxybuprocaïne.
